Europäisches Patentamt

European Patent Office

Office européen des brevets

(19)

(11) Publication number: **0 242 991**

**A2**

(12) **EUROPEAN PATENT APPLICATION**

(21) Application number: 87302419.4

(22) Date of filing: 20.03.87

(51) Int. Cl.4: **C12N 15/00** , C12Q 1/68 , A01H 1/02

The applicant has filed a statement in accordance with Rule 28 (4) EPC (issue of a sample only to an expert). Accession number(s) of the deposit(s): IVI - 10107, IVI - 10131.

(30) Priority: 20.03.86 US 841594
13.03.87 US 25514

(43) Date of publication of application:
28.10.87 Bulletin 87/44

(84) Designated Contracting States:
AT BE CH DE ES FR GB GR IT LI LU NL SE

(71) Applicant: **LUBRIZOL GENETICS INC.**
**3375 Mitchell Lane**
**Boulder Colorado 80301-2244(US)**

Applicant: **CORNELL RESEARCH FOUNDATION, INC.**
**East Hill Plaza**
**Ithaca, N.Y. 14850(US)**

(72) Inventor: **Tanksley Steven,D.,**
**404 Winston Court 1**
**Ithaca,New York 14853(US)**
Inventor: **Bernatzky,Robert,**
**519 East Buffalo 1**
**Ithaca,New York 14853(US)**

(74) Representative: **Fisher, Adrian John et al**
**CARPMAELS & RANSFORD 43 Bloomsbury Square**
**London WC1A 2RA(GB)**

(54) **Expanded tomato chromosome map and cDNA library.**

(57) This invention provides an expanded map of tomato chromosomes marked with the location of a large number of cDNA clones and isozyme markers. The cDNA clones are on deposit with In Vitro International, and may be used in conjunction with the map by finding linkages between desirable breeding characteristics of a tomato plant. After such linkages are established, they may be used to quickly determine the presence or absence of the desired characteristics in tomato plant and to introgress such desired characteristics into another tomato variety.

FIG. 2-1    FIG. 2-2

## EXPANDED TOMATO CHROMOSOME MAP AND cDNA LIBRARY

Field of the Invention

This invention lies in the field of genetic engineering using recombinant DNA clones, and in the field of plant breeding.

Background of the Invention

Genetic linkage maps have been developed for a wide variety of organisms and have been invaluable in the introgression of specific chromosomes or chromosome segments into various genetic backgrounds (Rick, C.M. and Khush, G.S. (1969) "Cytogenic explorations in the tomato genome", pp. 45-68, In: Genetics Lectures , Vol. I (R. Bogart ed.), Oregon State University Press, Corvalis; and C. Rhyne (1960) "Linkage studies in Gossypium II altered recombination values in linkage group of allotetraploid G. hirsutum L. as a result of transferred diploid species genes. Genetics 45:673-683). Localization of genes of interest can also be accomplished through linkage analysis with mapped markers as described by Patterson, E.B. (1982) "The mapping of genes by the use of chromosomal aberrations and multiple marker stocks", pp. 85-88, In: Maize for Biological Research (W.F. Sheridan, ed.) University Press, University of North Dakota, incorporated herein by reference.

Among higher plants, tomato (Lycopersicon esculentum) has one of the most extensive linkage maps. Marker loci have been used in tomato for the construction of chromosome substitution lines (Rick, C.M. (1969) "Controlled introgression of chromosomes of Solanum pennellii into Lycopersicon esculentum: segregation and recombination", Genetics 62:753-768; and Rick, C.M. (1971) "Some cytogenic features of the genome in diploid plant species", Stadler Symposia Vol. 1/2:153-174) and as an aid in the identification of genes involved in self-incompatibility (Tanksley, S.D. and Loaiza-Figueroa, F. (1985) "Genetic self incompatibility is controlled by a single major locus on chromosome 1 in Lycopersicon peruvianum, Proc. Nat'l. Acad. Sci. 82:5093-5096). The introgression of specific genes of economic importance such as male sterility (Tanksley, S.D., Rick, C.M. and Vallejos, C.E. (1984), "Tight linkage between a nuclear male-sterile locus and an enzyme marker in tomato", Theor. Appl. Genet. 68:109-113) and nematode resistance (Medina-Filho, H.P. (1980), "Linkage of Aps-1 , mi and other markers on chromosome 6", Rep. Tomato Genet. Coop. 30:26-28) has also been facilitated by the use of more easily selected linked marker loci.

Though the genetic map of tomato is well-populated with markers, it is, like most plant species, made up of primarily morphological mutant loci. In general, these morphological markers are not useful in selection programs for plant breeding since they are normally recessive and have undesirable phenotypes.

The development of electrophoresis and enzyme specific stains has expanded the map in tomato and has provided many new markers, the variants of which do not often have any noticeable effect on phenotype. These isozyme markers, though highly useful, are few in number and cover only a fraction of the tomato genome (Tanksley, S.D. and Rick, C.M. (1980), "Isozyme gene linkage map of the tomato: applications in genetics and breeding", Theor. Appl. Genet. 57 :161-170; Tanksley, S.D. (1985), "Enzyme coding genes in tomato (Lycopersicon esculentum), Isozyme Bulletin 18:43-45). For these reasons, it was desired to develop a more saturated linkage map in tomato based on and unique DNA sequences derived from messenger RNA (cDNA).

In recent years, unique DNA sequences corresponding to specific chromosomal sites have been used as genetic markers in humans (Botstein, D., White, R.L., Skolnick, M. and Davis, R. (1980), "Construction of a genetic linkage map in man using restriction fragment length polymorphisms", Am. J. Hum. Genet. 32:314-331; Jeffreys, A.J., Wilson, V. and Thein, S.L. (1985), "Hypervariable 'minisatellite' regions in human DNA", Nature 314:67-73; and White, R., Leppert, M., Bishop, D.T., Barker, D., Berkowitz, J., Brown, C., Callahan, P., Holm, T., Jerominski, L. (1985), "Construction of linkage maps with DNA markers for human chromosomes", Nature 313:101-105); and it has been suggested that this method be used in plants (Helentjaris, T.G., PCT Application published December 6, 1984, "Process for genetic mapping and cross-breeding thereon for plants"). With this approach, allelic variation is detected by first digesting DNA from the individuals being analyzed with a variety of restriction endonucleases. The resulting fragments are separated by electrophoresis and transferred to membranes. Allelic fragments are then identified by probing filters with radioactively-labelled, cloned, homologous sequences. Genetic variation detected in this manner has often been referred to as restriction fragment length polymorphism (RFLP). The number of RFLPs are virtually unlimited. They have no effect on phenotype, are codominant and are inherited in a Mendelian fashion (Botstein et al. 1980, supra).

Mapping of loci detected by RFLP's has advanced rapidly in humans and the information is finding application in prenatal diagnosis of a number of genetic disorders (Murray, J.M., Davies, K.E., Harper, P.S., Meredith, L., Mueller, C.R., Williamson, R. (1982), "Linkage relationships of a cloned DNA sequence on the short arm of the X chromosome to Duchenne muscular distrophy", Nature 300:69-70). While human geneticists have been rapidly developing maps using RFLP's (Solomon, F. and Goodfellow, P. (1983), "Human gene mapping rolls along", Nature 306:223-224), such mapping in higher plants, to this point, has been limited (Polans, N.O., Weeden, N.F. and Thompson, W.F. (1985), "Inheritance, organization and mapping of rbcs and cab multigene families in pea", Proc. Nat'l. Acad. Sci. 32:5083-5087).

R. Bernatzky et al. (1986), "Genetics of acton-related sequences in tomato", Theor. Appl. Genet. 72:314-321, showed a tomato linkage map including isozyme markers and acton-related sequences. Mapped cloned dNA sequences for tomato have been reported in R. Bernatzky et al. (1986), "Toward a Saturated Linkage Map in Tomato Based on Isozymes and Random cDNA Sequences", Genetics 112:887-898. The cDNA clones disclosed in said article were the basis of U.S. Patent Application No. 841,594, filed March 20, 1986, and incorporated herein by reference. The cDNA clones and expanded tomato chromosome map which form the subject of this application supplement the cDNA clones and map earlier disclosed.

R. Bernatzky et al. (1986), "Majority of random cDNA clones correspond to single loci in the tomato genome", Mol. Gen. Genet. 203:8-14, showed that most tomato cDNA clones derived by the methods of this invention map to only one place on the genome.

DNA markers in tomato will provide a basis for early screening procedures for many simply inherited characteristics as well as insight into the genetic organization of complex traits. Such screening procedures using cDNA markers is useful for the introgression of desirable traits into tomato varieties. A well-populated linkage map based on DNA sequences can also provide information about the evolution of plant genomes. Plant DNA fragments that hybridize to individual probes can be considered truly homologous and the genomic distribution of duplications and multi-gene family members can thus be compared among different genera.

## Summary of the Invention

This invention provides a map of tomato chromosomes marked with the location of a large number of cDNA clones and isozyme markers. The cDNA clones are on deposit with In Vitro International, and may be used in conjunction with the map by finding linkages between the clones and desirable, or observable breeding characteristics of a tomato plant. These linkages are established by means known to the art by comparing the percent recombination of the desired characteristic with the cDNA marker clones and designating the cDNA marker clone having the lowest percent recombination, preferably less than 30%, and more preferably less than 10%, with the desired characteristic, as being linked with the desired characteristic. The desired characteristic may be mapped on the tomato genome by placing it a calculated number of map units from previously mapped cDNA markers based on its recombination rate with these markers. Linkages between cDNA markers and observable characteristics may be used to quickly determine the presence or absence of the characteristics in particular tomato plants by testing for the presence of RFLP's characteristic of the linked markers. This ability to test genetic material without the necessity for growing up and breeding all plants is useful in breeding programs to introgress desired characteristics from one tomato variety into another.

## Description of the Figures

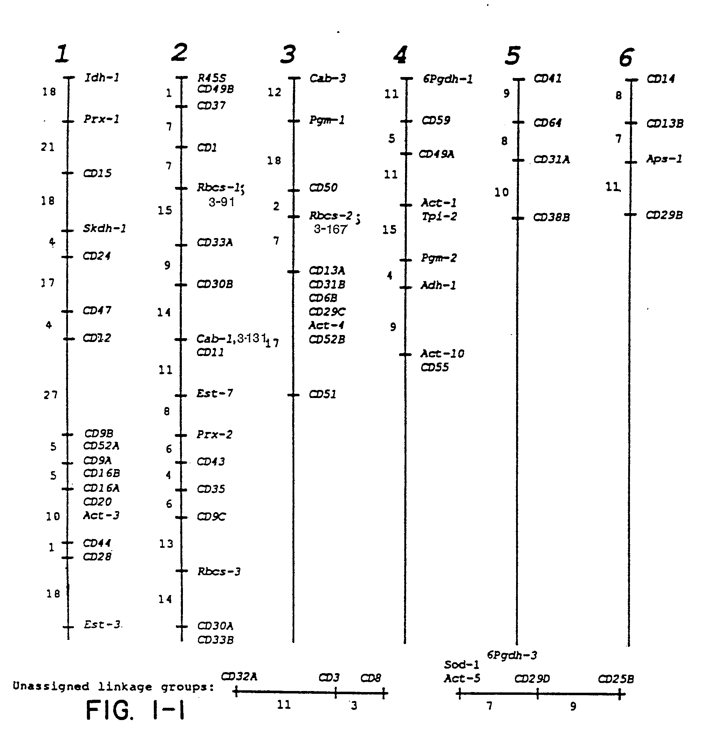

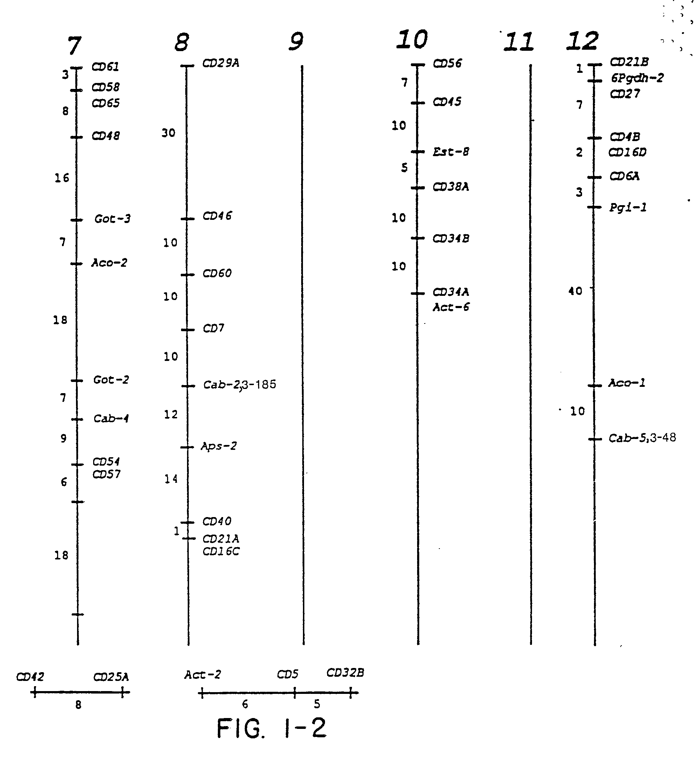

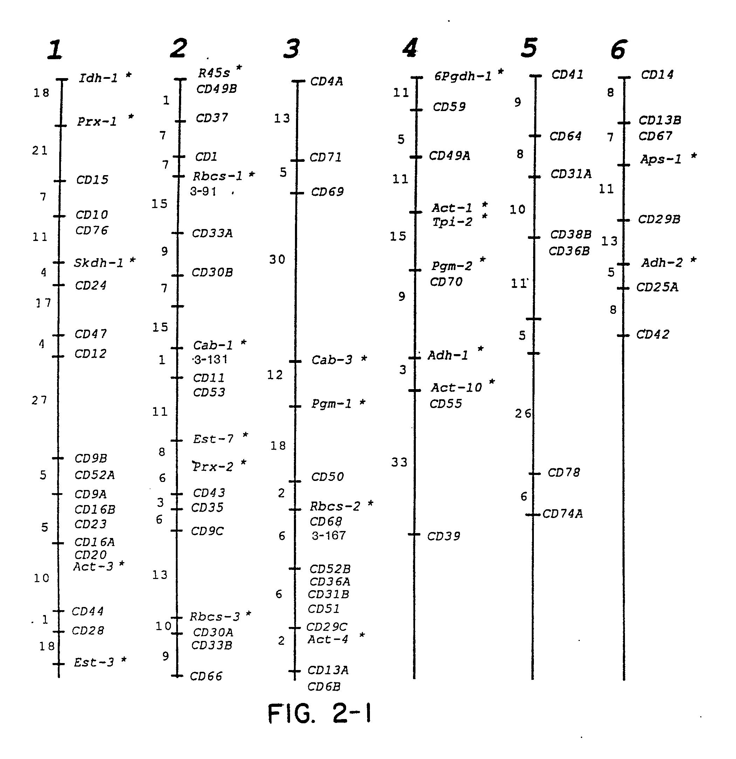

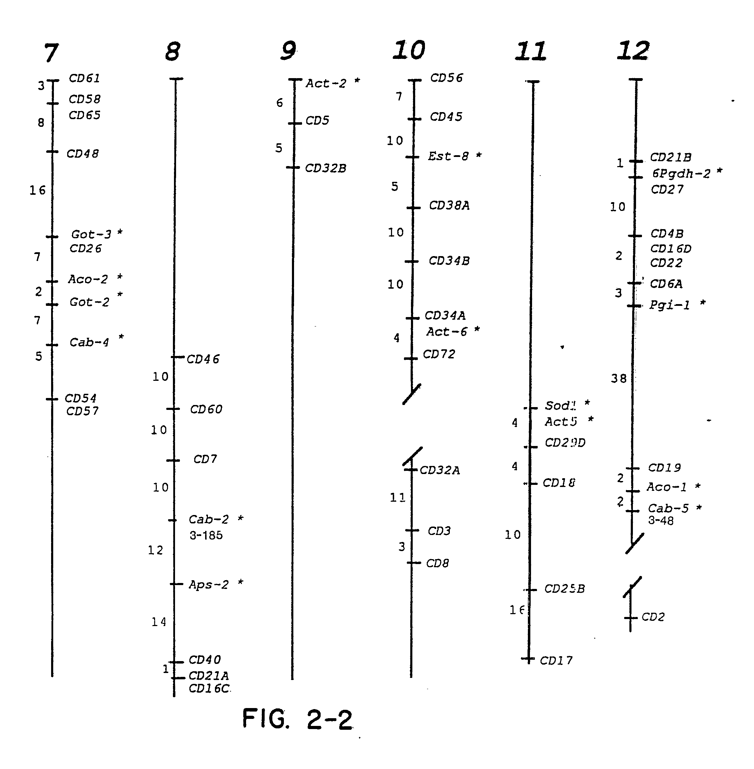

Figure 1 shows a tomato linkage map based on cDNA and isozyme markers as disclosed in U.S Patent Application No. 841,594. Figure 2 shows the expanded tomato linkage map of this invention. cDNA loci of this invention are designated by CD number or clone number. Alphabetical designations, A, B, C, D, following the CD number are used only to indicate first, second and multiple loci for the clone. The name of the clone with the alphabetical designation is considered equivalent to the name of the clone without the alphabetical designation. Other DNA markers are the large subunit of ribosomal RNA (R45s), the small subunit of ribulose bisphosphate carboxylase (Rbcs), the major chlorophyll a/b binding protein (Cab) and actin Act). All other markers are isozymes. Chromosome numbers are indicated at the top and the values along the side are map distances in cM. Linkage groups that did not show linkage with any of the mapped markers are indicated at the bottom of the figures. It will be apparent that this invention involves the chromosomal placement of previously unassigned clones as well as the mapping of new clones.

## Detailed Description of the Preferred Embodiments

This invention is based on the use of DNA restriction fragment length polymorphisms (RFLP's) to reveal differences in DNA taken from different plants.

DNA is isolated from a tomato plant and cut into fragments using a restriction enzyme by methods known in the art. A particular restriction enzyme cuts the DNA only at sites containing a specific nucleotide sequence, e.g. the restriction enzyme EcoRI cuts double stranded DNA only in the sequences GAATTC. Each restriction enzyme will cut the DNA of a particular plant into fragments of various lengths as specified by the distances between restriction enzyme recognition sites. Events such as insertion, deletion, and mutagenesis can alter the distance between restriction enzyme recognition sites.

As is known in the art, DNA fragments may be separated by size using gel electrophoresis. When it is desired to compare the DNA of two plants, the DNA samples of each are cut using the same restriction enzyme and the resulting fragments are separated according to size using electrophoresis. Many fragments from one variety may differ in length from their counterparts in another variety. Because any specific fragment represents a very small proportion of the total fragments, a sequence-specific probe must then be used to identify the specific homologous fragments in each DNA sample and permit comparison of fragment size.

This invention provides a unique set of cDNA probes for use in identifying corresponding DNA fragments from different tomato varieties. These probes were made, as described in the Examples hereof, by isolating mRNA from tomato variety VF36, preparation of a double-stranded copy DNA (cDNA) of the mRNA using reverse transcriptase, inserting the cDNA into bacterial plasmids, e.g. pBR322 and pUC8, and isolating clones harboring plasmids containing cDNA inserts (cDNA clones) larger than about 400-450 bp for further testing. All of these procedures are known in the art.

Individual cDNA clones were radioactively labeled to provide specific cDNA hybridization probes (probes). These cDNA probes will recognize substantially homologous sequences in any tomato variety. Tomato DNA fragments, after electrophoretic size separation, were transferred from the electrophoresis medium (typically an agarose gel) to a solid support (e.g. nitrocellulose or nylon membranes) such that the pattern resolved on the gel was preserved on the membrane. This membrane was incubated with radioactive probe during which time the probe hybridized to the specific corresponding DNA sequence. By observing the location of the probe on the membrane, it was possible to determine differences, or "length polymorphisms", between restriction fragments corresponding to the DNA of different tomato varieties. The procedure was repeated using different restriction enzymes and different probes until a number of restriction fragment length polymorphisms (RFLP's) by which the DNA of the two plants might be distinguished had been noted.

Only a few DNA fragments on the hybridization matrices showed homology to each cDNA probe, suggesting that the respective genes were present only once per haploid tomato genome. The majority of clones hybridized to different restriction fragments, indicating they each represented a different gene.

A set of unique cDNA probes, each identifying particular loci on the tomato genome, has been previously derived. These cDNA probes are listed in Table 1 hereof, and are named both according to their original designation during the cloning process and according to their loci. The entire library comprising 60 cDNA probes in plasmids is on deposit according to Budapest Treaty requirements at the In Vitro International Depository of 7885 Jackson Road, Ann Arbor, Michigan 48103, deposited March 19, 1986, entitled "Tomato Nuclear cDNA Clones", under Accession No. IVI-10107. The cDNA clones are designated as follows: CD15; CD24; CD47; CD12; CD9; CD52; CD16; CD20; CD44; CD28; CD49; CD37; CD1; CD33; CD30; CD11; CD43; CD35; CD50; CD13; CD31; CD6; CD29; CD51; CD59; CD55; CD41; CD64; CD38; CD14; CD61; CD58; CD65; CD48; CD54; CD57; CD62; CD46; CD60; CD7; CD40;; CD21; CD56; CD45; CD34; CD27; CD4; CD3; CD8; CD25; CD42; CD5; 3-91; 3-167; 3-185; 3-48; 3-131; CD2; CD32; and CD39.

A further set of unique cDNA probes, the subject of this patent application, was derived to expand the tomato chromosome map. the library of new probes, comprising 18 cDNA probes in plasmids, is on deposit according to Budapest Treaty requirements at In Vitro International Inc., 611P Hammonds Ferry Road, Linthicum, Maryland 21090, deposited March 16, 1987, entitled Tomato Nuclear cDNA Clones, under Accession No. IVI-0131. The cDNA clones are designated as follows: CD10, CD76, CD23, CD53, CD66, CD71, CD69, CD68, CD36, CD70, CD78, CD74, CD26, CD67, CD72, CD18, CD17 and CD19.

Using a number of different probes and restriction enzymes to analyze DNA from different plants, a DNA profile can be established for each plant, which is simply a compilation of the RFLP variants or alleles present in each variety. These profiles are used to differentiate plant varieties by comparing the RFLP's shown when one plant's DNA profile is compared with the DNA profile of the other.

This invention not only provides a method for identifying and distinguishing particular plant varieties, but because it was desired to use the cDNA clones in planned plant breeding programs, the position of the DNA sequence corresponding to each cDNA clone on the tomato chromosomes has been mapped. By the methods of this invention, individual traits or observable characteristics of tomato varieties can be linked with particular DNA markers, and this linkage used to transfer desired characteristics from one variety into another.

The cDNA clones are mapped as set forth below and as shown in Figure 2. The cDNA clones forming the subject matter of the present application are CD10, CD76, CD23, CD53, CD66, CD71, CD69, CD68, CD36, CD70, CD78, CD74, CD26, CD67, CD72, CD18, CD17, CD19, CD39 and CD2.

chromosome 1: Idh-1 - 18cM - Prx-1 - 21cM - CD15 - 7cM - CD10 and/or CD76 - 11cM - Skdh-1 - 4cM - CD24 - 17 cM - CD47 - 4cM - CD12 -27cM - CD98 and/or CD52A - 5cM - CD9A and/or CD16B and/or CD23 - 5cM - CD16A and/or CD20 and/or Act-3 - 10cM - CD44 -1cM - CD28 - 18cM - Est-3;

chromosome 2: R45s and/or CD49B - 1cM - CD37 - 7cM - CD1 - 7cM - Rbcs-1 and/or 3-91 - 15cM - CD33A - 9cM - CD30B - 22cM - Cab-1 and/or 3-131 - 1cM - CD11 and/or CD53 - 11cM - Est-7 - 8cM - Prx-2 - 6cM - CD43 - 3cM - CD35 - 6cM - CD9C - 13cM -Rbcs-3 - 10cM - CD30A and/or CD33B - 9cM - CD66;

chromosome 3: CD4A - 13cM - CD71 - 5cM - CD69 - 30cM - Cab-3 - 12cM -Pgm-1 - 18cM - CD50 - 2cM - Rbcs-2 and/or CD68 and/or 3-167 - 6cM - CD52B and/or CD36A and/or CD31B and/or CD51 - 6cM -CD29C and/or Act-4 - 2cM - CD13A and/or CD6B;

chromosome 4: 6Pgdh-1 - 11cM - CD59 - 5cM - CD49A - 11cM - Act-1 and/or Tpi-2 - 15cM - Pgm-2 and/or CD70 - 9cM - Adh-1 - 3cM - Act-10 and/or CD55 - 33cM - CD39;

chromosome 5: CD41 - 9cM - CD64 - 8cM - CD31A - 10cM - CD38B and/or CD36B - 42cM - CD78 - 6cM - CD74A;

chromosome 6: CD14 - 8cM - CD13B and/or CD67 - 7cM - Aps-1 - 11cM - CD29B - 13cM - Adh-2 - 5cM - CD25A - 8cM - CD42;

chromosome 7: CD61 - 3cM - CD58 and/or CD65 - 8cM - CD48 - 16cM - Got-3 and/or CD26 - 7cM - Aco-2 - 2cM - Got-2 - 7cM - Cab-4 - 5cM - CD54 and/or CD57;

chromosome 8: CD46 - 10cM - CD60 - 10cM - CD7 - 10cM - Cab-2 and/or 3-185 - 12cM - Aps-2 - 14cM - CD40 - 1cM - CD21A and/or CD16C;

chromosome 9: Act-2 - 6cM - CD5 - 5cM - CD32B;

chromosome 10: CD56 - 7cM - CD45 - 10cM - Est-8 - 5cM - CD38A - 10cM -CD34B - 10cM - CD34A and/or Act-6 - 4cM - CD72; CD32A -11cM - cD3 - 3cM - CD8;

chromosome 11: Sod1 and/or Act5 - 4cM - CD29D - 4cM - CD18 - 10cM - CD25B - 16cM - CD17;

chromosome 12: CD21B - 1cM - 6Pgdh-2 and/or CD27 - 10cM - CD4B and/or CD16D and/or CD22 - 2cM - CD6A - 3cM - Pgi-1 - 38cM - CD19 - 2cM - Aco-1 - 2cM - Cab-5 and/or 3-48; CD2

Clone 3-48 hybridizes to all cab sites, but preferentially to cab-5.

Applicants' invention consists of the creation and identification of the specific clones listed above as forming part of this invention, and also of the determination of the useful relationship of such clones to each other and to previously identified markers as indicated by their relative positions on the chromosome map. Other clones which may be prepared as hereinafter described and substituted for the above-listed underlined clones, and which map to the same sites on the chromosomes, are equivalent to the above clones.

Parents of two varieties (L. pennellii and L. esculentum) were crossbred and backcrossed, using L. pennelli as the recurrent parent, all as described in the Example, and the segregations of DNA markers and known genetic markers were evaluated in the BC1 progeny. These known genetic markers include isozyme markers known to the art and described, for example in Tanksley, S.D. (1983) "Molecular Markers in Plant Breeding" Plant Mol. Biol. 1:1 pp. 3-8, incorporated herein by reference, and cDNA markers as described in U.S. Patent Application No. 841,594, and R. Bernatzky et al. (1986) Genetics 112:887-898. The known tomato chromosome map including previously known cDNA and isozyme markers is shown in Figure 1. The expanded map including the cDNA markers of this invention is shown in Figure 2. Using established methods of genetic linkage analysis, the segregation data were used to map the positions on the tomato genome of the cDNA markers. This involved calculating the percent of the progeny in which a cDNA marker cosegregated with a known marker. Genetic map distance is defined by the percent recombination observed between two loci. For example, if a given DNA marker co-segregated with a previously mapped marker 100% of the time, there would be 0% recombination and thus, the DNA marker is 0 cM (centiMorgans; map units) from the previously mapped marker. A 10% recombination rate would indicate a 10cM map distance from the previous marker, etc. A discussion of known methods of genetic mapping using RFLP's and practical applications thereof is given in Beckmann, J.S. and Soller, M. (1983), "Restriction fragment length polymorphisms in genetic improvement: methodologies, mapping and costs", Theor. and Appl. Genetics 67:35-43; and Soller, M. and Beckmann, J.S. (1983), "Genetic polymorphism in varietal identification and genetic improvement", Theor. and Appl. Genetics 67:25-33, both of which are incorporated herein by reference. See also Burr, B., Evola, S.D., Burr F.A. and Beckmann, J.S. (1983), "The application of restriction fragment length polymorphisms to plant breeding", Genetic Engineering Principles and Methods (Setlow and Hollander, eds.) Vol. 5:45-49, also incorporated herein by reference.

The highly detailed map of this invention may be used to link phenotypic characteristics of a tomato plant with a particular marker or markers. To link an observable characteristic of a tomato plant with a cDNA marker clone or isozyme marker, the percent recombination of the observable trait with the marker is observed. Generally a recombination rate of less than 30% (indicating a map distance of 30cM or less), is considered a useful linkage, with a recombination rate of 10% or less being preferred.

More specifically, in linking an observable trait or characteristic such as high solids with a cDNA clone of this invention, a tomato variety having the observable characteristic and another variety not having the observable characteristic are selected. (An observable characteristic is one observable by visual inspection, physical or chemical testing or other means and includes RFLP's observed through the use of additional probes.) The two parental varieties are crossed and the F1 progeny either selfed or backcrossed to provide a population of individuals. These individuals are segregating for now known DNA markers and observable trait(s) of interest. The DNA is extracted from the segregating individuals and treated with a restriction enzyme, separated by gel electrophoresis, and then transferred to a hybridization support. After hybridization with a cDNA probe, one may determine which corresponding parental DNA fragments are present in each individual, the parental DNA profile having been previously determined. The process is repeated with additional cDNA probes to establish which parental chromosomes or parts thereof are present in each individual segregating progeny. Preferably, all the cDNA probes are used so that maximum segregation information may be developed. Isoenzyme markers may also be used to provide additional information. Additional restriction enzymes may be used as necessary to determine polymorphisms.

Each segregating individual is also scored for the desired phenotypic characteristic (e.g. high solids content). Genetic linkage analysis is then used to analyze the segregation of the observable trait(s) with known markers. DNA markers from the donor parent which recombine with the observable characteristic more than 50% of the time are considered to be unlinked to the DNA sequences controlling the observable characteristic. Markers which show 30% or less recombination may be considered to be usefully linked to the trait(s). Markers which show 10% or less recombination with the observable characteristic(s), are considered to be closely linked to the genes controlling the trait(s).

In practice, a limited subset of markers capable of revealing the parental chromosomes composition of each parent may be applied first. A subsequent set of markers for a specific chromosome would subsequently be used. For example, if markers on chromosome 5 show moderate linkage to high percent solids, then additional markers on chromosome 5 are selected and tested, until a marker which shows the least recombination with high percent solids is identified. The recombination frequencies are then used to map the genes controlling the observable characteristic.

Specifically, the method for determining an heritable association between an observable characteristic of a tomato plant and a cDNA clone of this invention comprises: (a) selecting a tomato plant having the observable characteristic and another tomato plant not having the observable characteristic as parents for crossbreeding; (b) extracting DNA from the parent plants and subjecting said DNA to treatment with a restriction enzyme to obtain DNA fragments; separating the fragments from each plant on a neutral gel by electrophoresis and transferring the electrophoresed fragments to a solid support to obtain a hybridization matrix for each plant; (c) hybridizing the matrices of step (b) with a cDNA clone selected from the cDNA clones of this invention marking a chromosome or unassigned linkage group which might contain DNA corresponding to said observable trait (for example, prior experiments may indicate or rule out certain chromosomes as containing DNA controlling the trait); (d) comparing the hybridization pattern from each parent to determine the presence of polymorphisms; (e) repeating steps (b) - (d) utilizing at least one additional cDNA clone selected from those marking other chromosomes or unassigned linkage groups which might contain DNA corresponding to the said observable characteristic and/or using additional restriction enzymes as necessary until polymorphisms have been identified by which the parents may be distinguished from each other by at least one difference on each chromosome or unassigned linkage group which might contain DNA corresponding to the said observable characteristic; (f) crossing the parent plants to obtain an F1 generation and selfing or backcrossing the F1 generation to obtain a segregating generation containing individuals having the observable characteristic; (g) selecting and scoring a statistically significant number of segregating individuals, preferably at least about 40, for the percent presence of the observable characteristic; (h) extracting DNA from the segregating individuals of step (g) and following the procedures of steps (b) through (e) to obtain hybridized matrices for each individual showing polymorphisms when compared to the matrices of the parent not having the observable characteristic; (i) identifying cDNA clones which are located within 10 map units (10 cM) of genes conferring the observable characteristic; (j) if no such cDNA clones are identified, identifying cDNA clones which are located less than 30 map units (cM) from genes controlling the observable characteristic; (k) selecting additional cDNA clones from the cDNA clones of this invention, said cDNA clones having map positions spaced less than 30 map units (cM) from the cDNA clones of step (j), and hybridizing said clones with the DNA of segregating individuals of step (h); (l) identifying cDNA clones which are located within 10 map units (10cM) from genes controlling the observable characteristic; (m) if no such cDNA clones are identified, selecting additional cDNA clones from the cDNA clones of this invention, said cDNA clones having map positions spaced closer to previously tested cDNA clones having high percent identity than those having a lower percent identify, hybridizing said clones with the DNA of segregating individuals of step (h) and identifying cDNA clones which are located less than 10 map units from genes controlling the observable characteristic; (n) if no such cDNA clones are identified, repeating step (m) until such clones have been identified.

Isozyme and other markers can be utilized in addition to cDNA clones to link with heritable characteristics by correlating the presence or occurrence of each isozyme occurring in the parent having the observable characteristic with the percent presence of the observable characteristic in the segregating generation individuals.

DNA controlling the observable characteristic may be located on the chromosome map by placing it the number of map units from specific markers corresponding to its percent recombination with such markers.

The cDNA clones of this invention may be used to develop additional or substitute clones mapping to the. same or contiguous regions (0 map units as defined by recombination rates) of the genome. For example, a phage or other clone library is probed with a cDNA clone of this invention and additional clones having sufficient homology to the cDNA clone to hybridize therewith are identified. Such newly-identified clones may be subcloned, utilizing restriction enzymes, and the newly-identified clones or fragments thereof are tested for their recombination rate with the previously mapped cDNA clones or linked characteristics. Those showing a 0-10% recombination rate are substitute clones equivalent to the named clones of this invention.

Hybridization conditions utilized in the procedures described herein are as set forth in the Example. Hybridization conditions equivalent to those described herein may be employed by those skilled in the art utilizing well-known, published equations, for example as described in Nucleic Acid Hybridization, A Practical Approach, Hames, B.D. and Higgins, S.J., eds (1985) IRL Press, Oxford, incorporated herein by reference.

Once a linkage between an observable characteristic and a marker has been established, any individual plant may be identified at an early stage of growth for the presence of the gene controlling that characteristic by noting the appearance of marker known to be linked with the characteristic. Consequently, a desired characteristic may be introgressed from one tomato variety into a second tomato variety having other desirable characteristics. For example, moving a trait such as Fusarium resistance from a wild tomato variety having otherwise undesirable fruit characteristics into a domestic variety can be facilitated by the availability of a marker which is closely linked to Fusarium resistance. As in the example provided above, a DNA marker which is closely linked to the genes controlling Fusarium resistance must first be identified. Segregating progeny resulting from a cross between the Fusarium resistance donor and the recipient are tested for the presence of this marker. Only those individuals carrying the marker need be saved. For example, if the desirable trait is due to one gene, 75% of the individuals could be discarded. In the process, additional markers are used to evaluate the extent to which each individual carrying the Fusarium resistance gene represents each parental genome. Those individuals which carry the Fusarium resistance marker and most closely represent the desirable parental genome would be retained for additional cycles of backcrossing with the desirable domestic variety. The backcrossing and screening is continued until individuals homozygous for the desired characteristic but otherwise having a DNA profile very close to the domestic parent are obtained. The key advantage of this approach is that it allows the breeder to converge upon the desirable genotype(s) rapidly with a minimum of field testing.

Specifically, a desired characteristic may be transferred from a tomato plant into a second tomato variety by: (a) linking the desired characteristic with a marker selected from the markers of this invention, isozyme or cDNA markers of Fig. 2 or other markers; (b) probing DNA of the second tomato variety with cDNA of this invention to determine its DNA profile; (c) crossbreeding a tomato plant having the desired characteristic with a plant of the second tomato variety to obtain an F1 generation; (d) screening F1 individuals for being homozygous for the desired characteristic using the linked marker; (e) probing the DNA of the F1 individuals having the desired characteristic with cDNA of this invention to determine their DNA profiles; (f) selecting F1 individuals having the desired characteristic and otherwise having DNA profiles most similar to that of the parent of the second tomato variety for breeding; (g) backcrossing the selected individuals of step (f) with a parent of the second tomato variety to produce an BC1 generation; (h) screening BC1 individuals for the desired characteristic using the linked marker; (i) probing the DNA of the BC1 individuals having the desired characteristic with cDNA of this invention to determine their DNA profiles; (j) selecting BC1 individuals having the desired characteristic and otherwise having DNA profiles similar to that of the parent of the second tomato variety; and (k) repeating steps (g) - (i) with BC2 and subsequent generations until individuals homozygous for the desired characteristic and having the desired degree of similarity to the parent of the second tomato variety are produced.

An important application of the ability to use the clones of this invention to test genetic material for the presence or absence of DNA controlling particular traits is in the introgression of additional disease resistance genes into plant varieties already having other genes controlling resistance to the same disease. Overall resistance to a disease is greater in plants having several different genes controlling such resistance, but is is very difficult to test for the presence of multiple disease resistance genes in an actual plant because the presence of one resistance gene masks identification of resistance caused by additional genes. The use of the mapped markers of this invention to identify such additional resistance genes enables plant breeders to easily select for their presence and breed them into already resistant varieties.

The cDNA markers, chromosome map, and screening method of this invention are useful in a number of other applications which will be apparent to those skilled in the art. For example, these markers and methods may be used in estimation of ontcrossing rates, identification of sexual and parasexual hybrids, identification of breeding stock in perennial nurseries, measurement of genetic variability, determination of genetic purity of hybrid seed lots, identification of infringement of patents covering plant varieties and recombinant DNA, and identification of haploid-derived plants from anther culture.

The following example illustrates the development and use of the cDNA clones of this invention.

## Example

Plant materials. Segregating populations were obtained from crosses between inbred lines of Lycopersicon esculentum (LA1500) and L. pennelli (LA716). A backcross to L. pennellii (staminate parent) and a BC1 population of 46 plants each were analyzed. The two parental lines had allelic differences at the following isozyme loci: Aco-1, Aco-2, Aps-1, Aps-2, Pgm-1, Pgm-2,6Pgdh-2, Skdh-1, Prx-1, Prx-2, Prx-4, Pgi-1, Tpi-2,sod-1, Sod-2, Est-3 Est-7, and Est-8. Except for Sod-1, Sod-2 and Est-8, all of the isozymes had been previously mapped (Tanksley, S.D. (1985) "Enzyme coding genes in tomato (Lycopersicon esculentum)", Isozyme Bull. 18 :43-45, incorporated herein by reference. Linkage analysis of isozyme markers in relation to genomic sequences homologous to the major chlorophyll a/b binding protein (Cab), the small subunit of ribulose bisphosphate carboxylase (Rbcs) and the large subunit of ribosomal RNA (R45s) have been reported by Vallejos et al. (1986) supra, incorporated herein by reference, and in this work provided additional segregating loci with which to map the random cDNA markers of this invention. In addition, the cDNA markers of U.S. Patent Application No. 841,594 and R. Bernatzky et al. (1986) Genetics 112:887-898 were used to map the cDNA markers of this invention.

DNA extractions: DNA was extracted from plant material by a modified procedure of Murray and Thompson (1980), supra, as described in U.S. Application Serial No. 841,594, and R. Bernatzky et al. (1986), Theor. Appl. Genet. 72:314-321.

mRNA isolation and cDNA cloning: Total polyadenylated leaf mRNA was isolated and cloned into pBR322 as described in R Bernatzky et al. (1986) Mol. Gen. Genet. 203:8-14.

Restriction digests, electrophoresis, Southern blotting and hybridizations: DNA from parental lines and their progeny were each digested with the following restriction enzymes: Dra I, HindIII (New England BioLabs), EcoRI, EcoRV, SstI, PstI and Xba I (Bethesda Research Labs). Electrophoresis of plant DNA, Southern blotting, hybridization and nick translation of probes were as described R. Bernatzky et al. (1986) Genetics 112 :887-898.

cDNA screening: Random cDNA clones were screened for insert size and those clones with inserts greater than 450 base pairs (bp were used as probes. The parental DNAs were digested with various restriction enzymes, electrophoresed, blotted and probed with the cDNAs to determine which enzymes produced fragment length polymorphisms. The appropriate enzymes were then used on progeny DNA. For the backcross progeny, L. pennellii was used as the recurrent parent such that only the L. esculentum fragments were segregating as present or absent. The BC1 progeny segregated into the expected three genotypes for each locus with occasional skewing towards L.pennellii homozygotes.

Linkage analysis: Two-way contingency tests for independent assortment of isozyme markers and cDNA sequences based on chi-square analysis were accomplished using the SAS Proc Freq software program on an Amdahl 470/V5 computer. Loci were considered to be independent if the chi-square probability level was greater than 0.05. Map distances (cM) were calculated based on the maximum likelihood equations of Allard, R.W. (1954), "Formulas and tables to facilitate the calculation of recombination values in heredity", Hilgardia 24:235-278, incorporated herein by reference.

Table 1:  Summary of cDNA clones, the number of loci per clone and their
assigned chromosomes.

| Clone | Locus designation | Chromosomal assignment | Clone | Locus designation | Chromosomal assignment |
|-------|-------------------|------------------------|-------|-------------------|------------------------|
| 2-13 | CD1 | 2 | 3-176 | CD34A | 10 |
| 3-17 | CD2 | | | CD34B | 10 |
| 3-14 | CD3 | | 3-231 | CD35 | 2 |
| 2-23 | CD4A | | 3-288 | CD37 | 2 |
| | CD4B | 12 | 3-275 | CD38A | 10 |
| 2-14 | CD5 | 10 | | CD38B | 5 |
| 3-6 | CD6A | 12 | 3-287 | CD39 | |
| | CD6B | 3 | 3-249 | CD40 | 8 |
| 3-13 | CD7 | 8 | 3-217 | CD41 | 5 |
| 2-24 | CD8 | | 3-218 | CD42 | |
| 2-21 | CD9A | 1 | 2-17 | CD43 | 2 |
| | CD9B | 1 | 3-4 | CD44 | 1 |
| | CD9C | 2 | 3-10 | CD45 | 10 |
| 3-50 | CD11 | 2 | 3-16 | CD46 | 8 |
| 3-31 | CD12 | 1 | 3-27 | CD47 | 1 |
| 3-87 | CD13A | 3 | 3-38 | CD48 | 7 |
| | CD13B | 6 | 3-44 | CD49A | 4 |
| 3-41 | CD14 | 6 | | CD49B | 2 |
| 3-82 | CD15 | 1 | 3-81 | CD50 | 3 |
| 3-95 | CD16A | 1 | L4 | CD51 | 3 |
| | CD16B | 1 | L1 | CD52A | 1 |
| | CD16C | 8 | | CD52B | 3 |
| | CD16D | 12 | L3 | CD54 | 7 |
| 3-109 | CD20 | 1 | L2 | CD55 | 4 |
| 3-93 | CD21A | 8 | L5 | CD56 | 10 |
| | CD21B | 12 | L9 | CD57 | 7 |
| 3-111 | CD24 | 1 | L17 | CD58 | 7 |
| 3-99 | CD25A | | L16 | CD59 | 4 |
| | CD25B | | L13 | CD60 | 8 |
| 3-132 | CD27 | 12 | L18 | CD61 | 7 |
| 3-152 | CD28 | 1 | L20 | CD62 | 7 |
| 3-155 | CD29A | | L22 | CD64 | 5 |
| | CD29B | 6 | L10 | CD65 | 7 |
| | CD29C | 3 | | | |
| | CD29D | | 3-91, | Rbc-1 | 2 |
| 3-140 | CD30A | 2 | 3-167 | Rbc-2 | 3 |
| | CD30B | 2 | | Rbc-3 | 2 |
| 3-126 | CD31A | 5 | | | |
| | CD31B | 3 | 3-131, | Cab-1 | 2 |
| 3-159 | CD32A | | 3-185 | Cab-2 | 8 |
| | CD32B | 10 | | Cab-3 | 3 |
| 3-190 | CD33A | 2 | | Cab-4 | 7 |
| | CD33B | 2 | 3-48 | Cab-5 | 12 |

A sample restriction enzyme survey showed that the restriction enzymes EcoRV and BstNI are suitable to distinguish the two parents.

An example of the linkage analysis used is set forth in R. Bernatzky et al. (1986) Genetics 112 :887-898. Although the deduced gene order could usually be derived from pairwise linkage comparisons alone, three point linkage tests were always employed to confirm the linear order of linked markers.

cDNA markers have been mapped to all of the tomato chromosomes (Figure 2). Chromosomes 1, 2 and 12 are very well marked. For example, the distance between the markers on chromosome 2, including isozymes and DNA fragments, averages approximately 8 cM and these markers cover a total map distance of 128 cM. The total presently-mapped isozyme and DNA markers cover approximately 852 cM between markers and have the ability to detect linkage for at least an additional 10 cM beyond each end. These markers, then are able to detect linkage over 91% of the tomato genome based on an estimate of 1200 cM for the entire genome.

Chromosome 5 did not have any segregating isozyme markers associated with it. However, a stock with morphological mutants that have been assigned to this chromosome was employed. DNA was isolated from 20 individuals of an F2 population (L. esculentum x L. pennellii) that was segregating for two markers on chromosome 5 (af and tf) and the various unassigned linkage groups were tested against these markers. cDNA marker CD41 showed tight linkage with tf such that the linkage group comprised of CD38B, CD31A and CD41 could be assigned to chromosome 5.

Assignment of markers to chromosomes 9 and 11 was done through the use of trisomics. Clones hybridizing with stronger signals to genomic DNA from plants trisomic for each chormosome were assigned to that chromosome.. The use of trisomics for assignment of (isozyme) markers is known to the art. See S.D. Tanksley (1979), "Linkage, chromosomal association and expression of ADH1 and Pgm2 in tomato", Biochem. Gen. 17:1159-1167.

Most of the cDNA fragments were found to correspond to single chromosomal locations. A few of the clones, however, are represented by sequences arranged as two, three or four loci (Table 1). Of these multi-locus clones, the majority show genetic independence among members, that is, the loci are dispersed throughout the genome. For example, a restriction enzyme survey of parental DNAs probed with 3-275 (CD38A and B) as well as progeny DNA from the BC1 population shows this clone hybridizes to two fragments in both parental DNAs, and as indicated by the progeny DNA, the two fragments represent two loci which are genetically independent. There are a few exceptions to the independence of multi-locus clones. Two clones, 2-21 (CD9) and 3-176 (CD34), both of which hybridized to fragments at two loci, have linkage values of 5 and 10 cM between duplicate loci, respectively (Figure 1, chromosome 1 and 10). A number of other multi-locus clones have members that map to the same chromosome though they are more than 50 cM apart. As can be seen on chromosome 2, clones that represent CD30, CD33 and Rbcs are linked in two clusters, suggesting the duplication of a rather large segment.

As discussed above, the map distances given herein in Figures 1 and 2 are statistically derived. It should be understood that variations in map distances of 5 to 10 cM are not statistically significant, and are within the scope of equivalents of this invention.

The foregoing Examples are intended to illustrative rather than limiting to the scope of these inventions.

## Claims

1. A cDNA library corresponding to tomato DNA comprising at least two clones selected from the group consisting of CD10, CD76, CD23, CD53, CD66, CD71, CD69, CD68, CD36, CD70, CD78, CD74, CD26, CD67, CD72, CD18, CD17, CD19, DC39 and CD2.

2. The cDNA library of claim 1 wherein said two clones are selected from the same chromosome.

3. The cDNA library of claim 1 containing all the clones listed in claim 1.

4. A cDNA clone selected from the group consisting of CD10, CD76, CD23, CD53, CD66, CD71, CD69, CD68, CD36, CD70, CD78, CD74, CD26, CD67, CD72, CD18, CD17, CD19, CD39 and CD2.

5. A plasmid containing a cDNA clone of claim 4.

6. The plasmid of claim 5 comprising pBR322 or pUC8.

7. A chromosome map useful for determining the original position or positions of a tomato DNA fragment on the tomato chromosome comprising a representation of at least one chromosome with markers spaced therealong in sequential order with spacing given in map units (cM) as follows:

chromosome 1: Idh-1 - 18cM - Prx-1 - 21cM - CD15 - 7cM - CD10 and/or CD76 - 11cM - Skd-1 - 4cM - CD24 - 17cM - CD47 - 4cM -CD12 - 27cM - CD9B and/or CD52A - 5cM - CD9A and/or CD16B and/or CD23 - 5cM - CD16A and/or CD20 and/or act-3 - 10 cM -.CD44 - 1cM - CD28 - 18cM - Est-3;

chromosome 2: R45s and/or CD49B - 1cM - CD37 - 7cM - CD1 - 7cM -Rbcs-1 and/or 3-91 - 15cM - CD33A - 9cM - CD30B - 22cM - Cab-1 and/or 3-131 - 1cM - CD11 and/or CD53 - 11cM - Est-7 - 8cM -Prx-2 - 6cM - CD43 - 3cM - CD35 - 6cM - CD9C - 13cM - Rbcs-3 -10cM - CD30A and/or CD33B - 9cM - CD66;

chromosome 3: CD4A - 13cM - CD71 - 5cM - CD69 - 30cM - Cab-3 -12cM - Pgm-1 - 18cM - CD50 - 2cM - Rbcs-2 and/or CD68 and/or 3-167 - 6cM - CD52B and/or CD36A and/or CD31B and/or CD51 - 6cM -CD29C and/or Act-4 - 2cM - CD13A and/or CD6B;

chromosome 4: 6Pgdh-1 - 11cM - CD59 - 5cM - CD49A - 11cM - Act-1 and/or Tpi-2 - 15cM - Pgm-2 and/or CD70 - 9cM - Adh-1 - 3cM -Act-10 and/or CD55 - 33cM - CD39;

chromosome 5: CD41 - 9cM - CD64 - 8cM - CD31A - 10cM - CD38B and/or CD36B - 42cM - CD78 - 6cM - CD74A;

chromosome 6: CD14 - 8cM - CD13B and/or CD67 - 7cM - Aps-1 - 11cM - CD29B - 13cM - Adh-2 - 5cM - CD25A - 8cM - CD42;

chromosome 7: CD61 - 3cM - CD58 and/or CD65 - 8cM - CD48 - 16cM -Got-3 and/or CD26 - 7cM - Aco-2 - 2cM - Got-2 - 7cM - Cab-4 -5cM - CD54 and/or CD57;

chromosome 8: CD46 - 10cM - CD60 - 10cM - CD7 - 10cM - Cab-2 and/or 3-185 - 12cM - Aps-2 - 14cM - CD40 - 1cM - CD21A and/or CD16C;

chromosome 9: Act-2 - 6cM - CD5 - 5cM - CD32B;

chromosome 10: CD56 - 7cM - CD45 - 10cM - Est-8 - 5cM - CD38A -10cM - CD34B - 10cM - CD34A and/or Act-6 - 4cM - CD72; CD32A -11cM - cD3 - 3cM - CD8;

chromosome 11: Sod1 and/or Act5 - 4cM - CD29D - 4cM - CD18 - 10cM -CD25B - 16cM - CD17;

chromosome 12: CD21B - 1cM - 6Pgdh-2 and/or CD27 - 10cM - CD4B and/or CD16D and/or CD22 - 2cM - CD6A - 3cM - Pgi-1 - 38cM -CD19 - 2cM - Aco-1 - 2cM - Cab-5 and/or 3-48; CD2

8. The map of claim 7 in combination with at least one of the cDNA clones named therein.

9. A method for linking an observable characteristic of a tomato plant with a cDNA marker clone of claim 1 comprising comparing the percent recombination of the observable characteristic with the cDNA marker clones of claim 1 and designating the cDNA marker clone having the lowest percent recombination with the observable characteristic as being linked with the observable characteristic.

10. The method of claim 9 wherein the cDNA clones displaying less than 30% recombination with the observable characteristic are designated as linked with said characteristic.

11. A method for identifying a substitute DNA marker comprising probing a DNA library with a cDNA marker of claim 1 to identify library DNA substantially homologous to said cDNA marker, observing the recombination rate of said library DNA or a fragment thereof with said cDNA marker or an observable characteristic linked thereto, and designating library DNA having a 0-10% recombination rate as a substitute DNA marker.

12. A method for determining an heritable association between an observable characteristic of a tomato plant and a cDNA clone of claim 1 comprising:

(a) selecting a tomato plant having the observable characteristic and another tomato plant not having the observable characteristic as parents for crossbreeding;

(b) extracting DNA from the parent plants and subjecting said DNA to treatment with a restriction enzyme to obtain DNA fragments; separating the fragments from each plant on a neutral gel by electrophoresis and transferring the electrophoresed fragments to a solid support to obtain a hybridization matrix for each plant;

(c) hybridizing the matrices of step (b) with a cDNA clone selected from the cDNA clones of claim 1 marking a chromosome or unassigned linkage group which might contain DNA corresponding to said observable characteristic;

(d) comparing the hybridization pattern from each parent to determine the presence of polymorphisms;

(e) repeating steps (b) - (d) utilizing at least one additional cDNA clone selected from those marking other chromosomes which might contain DNA corresponding to the said observable characteristic and/or using additional restriction enzymes as necessary until polymorphisms have been identified by which the parents may be distinguished from each other by at least one difference on each chromosome or unassigned linkage group which might contain DNA corresponding to the said observable characteristic;

(f) crossing the parent plants to obtain an F1 generation and selfing or backcrossing the F1 generation to obtain a segregating generation containing individuals having the observable characteristic;

(g) selecting and scoring a statistically significant number of segregating individuals for the percent presence of the observable characteristic;

(h) extracting DNA from the segregating individuals of step (g) and following the procedures of steps (b) through (e) to obtain hybridized matrices for each individual showing polymorphisms when compared to the matrices of the parent not having the observable characteristic;

(i) identifying cDNA clones which are located within 10 map units (10 cM) of genes conferring the observable characteristic;

12

(j) if no such cDNA clones are identified, identifying cDNA clones which are located less than 30 map units·(cM) from genes controlling the observable characteristic;

(k) selecting additional cDNA clones from the cDNA clones of claim 1, said cDNA clones having map positions spaced less than 30 map units (cM) from the cDNA clones of step (j), and hybridizing said clones with the segregating generation DNA of step (h);

(l) identifying cDNA clones which are located within 10 map units (10cM) from genes controlling the observable characteristic;

(m) if no such cDNA clones are identified, selecting additional cDNA clones from the cDNA clones of claim 1, said cDNA clones having map positions spaced closer to previously tested cDNA clones having high percent identity than those having a lower percent identity, hybridizing said clones with the DNA of segregating individuals of step (h) and identifying cDNA clones which are located less than 10 map units from genes controlling the observable characteristic.

(n) if no such cDNA clones are identified, repeating step (m) until such clones have been identified.

13. The method of claim 9 where additional markers are utilized in addition to said cDNA clones and the presence or occurrence of each isozyme occurring in the parent having the observable characteristic is correlated with the percent presence of the observable characteristic in the segregating generation individuals.

14. The method of claim 9 including locating DNA controlling the observable characteristic on a chromosome map.

15. A method for screening a tomato plant for the presence of an observable characteristic comprising:

(a) linking the observable characteristic with a marker selected from the cDNA clones of claim 1; and

(b) testing the DNA of the tomato plant for the presence of RFLP's characteristic of the selected marker.

16. A method for transferring a desired characteristic from a tomato plant into a second tomato variety comprising:

(a) linking the desired characteristic with a previously mapped marker;

(b) probing DNA of the second tomato variety with cDNA of claim 1 to determine its DNA profile;

(c) crossbreeding a tomato plant having the desired characteristic with a plant of the second tomato variety to obtain an F1 generation;

(d) screening F1 individuals for being homozygous for the desired characteristic using the linked marker;

(e) probing the DNA of the F1 individuals having the desired characteristic with cDNA of claim 1 to determine their DNA profiles;

(f) selecting F1 individuals having the desired characteristic and otherwise having DNA profiles most similar to that of the parent of the second tomato variety for breeding;

(g) backcrossing the selected individuals of step (f) with a parent of the second tomato variety to produce an BC1 generation;

(h) screening BC1 individuals for the desired characteristic using the linked marker;

(i) probing the DNA of the BC1 individuals having the desired characteristic with cDNA of claim 1 to determine their DNA ]profiles;

(j) selecting BC1 individuals having the desired characteristic and otherwise having DNA profiles similar to that of the parent of the second tomato variety; and

(k) repeating steps (g) - (i) with BC2 and subsequent generations until individuals homozygous for the desired characteristic and having the desired degree of similarity to the parent of the second tomato variety are produced.

**1**

Idh-1
18
Prx-1
21
CD15
18
Skdh-1
4
CD24
17
CD47
4
CD22
27
CD9B
5   CD52A
CD9A
5   CD16B
CD16A
CD20
10  Act-3
1   CD44
CD28
18
Est-3

**2**

R45S
1   CD49B
CD37
7
CD1
7
Rbcs-1;
15      3-91
CD33A
9
CD30B
14
Cab-1,3·131
17
CD11
11
Est-7
8
Prx-2
6
CD43
4
CD35
6
CD9C
13
Rbcs-3
14
CD30A
CD33B

**3**

Cab-3
12
Pgm-1
18
CD50
2
Rbcs-2;
7        3-167
CD13A
CD31B
CD6B
CD29C
Act-4
CD52B
CD51

**4**

6Pgdh-1
11
CD59
5
CD49A
11
Act-1
Tpi-2
15
Pgm-2
4
Adh-1
9
Act-10
CD55

**5**

CD41
9
CD64
8
CD31A
10
CD38B

**6**

CD14
8
CD13B
7
Aps-1
11
CD29B

Unassigned linkage groups:

CD32A      CD3      CD8
        11        3

Sod-1    6Pgdh-3
Act-5    CD29D        CD25B
      7          9

## FIG. I-I

**7**

3 — CD61
— CD58
8 — CD65
— CD48

16 —

— Got-3
7 —
— Aco-2

18 —

— Got-2
7 —
— Cab-4
9 —
— CD54
— CD57
6 —

18 —

**8**

— CD29A

30 —

— CD46
10 —
— CD60
10 —
— CD7
10 —
— Cab-2,3-185
12 —
— Aps-2
14 —

— CD40
1 — CD21A
— CD16C

**9**

**10**

— CD56
7 —
— CD45
10 —
— Est-8
5 —
— CD38A
10 —
— CD34B
10 —
— CD34A
Act-6

**11**

**12**

1 — CD21B
— 6Pgdh-2
7 — CD27
— CD4B
2 — CD16D
— CD6A
3 —
— Pgi-1

40 —

— Aco-1
10 —
— Cab-5,3-48

CD42     CD25A
+———————+
8

Act-2    CD5    CD32B
+————+——+
6    5

## FIG. 1-2

FIG. 2-1

FIG. 2-2